# EUROPÄISCHE PATENTANMELDUNG

(11) **EP 1 700 617 A1**
(43) Veröffentlichungstag der Anmeldung: **13.09.2006**
(21) Anmeldenummer: 06004127.4
(22) Anmeldetag: 01.03.2006
(51) Int. Cl.: A61Q 7/00, A61K 8/49, A61K 8/97, A61P 17/04

(54) **Mittel zur Aktivierung der Haarwurzeln und Verwendung des Mittels**

(30) Priorität: 02.03.2005 DE 102005010142
(71) Anmelder: Dr. Kurt Wolff GmbH & Co. KG, 33611 Bielefeld (DE)
(72) Erfinder: Salmina, Manuela, 33098 Paderborn (DE); Klenk, Adolf, 33415 Verl (DE)
(74) Vertreter: Vièl, Christof

(57) **Zusammenfassung**

Die Erfindung betrifft ein Mittel zur Aktivierung der Haarwurzeln und Verwendung des mittels.

Um bei einem Mittel zur Aktivierung der Haarwurzeln die Wirkung der "östrogenen" Pflanzenextrakte noch zu verstärken wird im Rahmen der Erfindung vorgeschlagen, dass das Mittel aus einer Kombination von Coffein und einem Pflanzenextrakt mit phytoöstrogenen Eigenschaften besteht.

Überraschenderweise wurde gefunden, dass sich die den Haarwuchs fördernden Eigenschaften durch den Zusatz von Coffein deutlich steigern ließen. Coffein ist eine Substanz, die aufgrund der geringen Molekülgröße in die Haut penetrieren kann. Interessanterweise ist der Haarwuchs kräftigende Effekt durch die Mischung der beiden Wirkprinzipien besser als er durch Einzelkomponenten zu erwarten wäre.

## Beschreibung

Die Erfindung betrifft ein Mittel zur Aktivierung der Haarwurzeln und Verwendung des Mittels.

Die Behandlung von androgenetisch bedingtem Haarausfall ist nach wie vor ein ungelöstes Problem. Durch eine genetische Veranlagung ist festgelegt, dass ab einem bestimmten Zeitpunkt die Haarfollikel im fronto-parietalen und im zentralen Sektor der Kopfhaut eine Überempfindlichkeit gegen Testosteron und seinen Metaboliten 5-alpha-Dihydrotestosteron (5a-DHT)entwickeln. Durch eine Signalübertragung des 5a-DHT Rezeptors wird das Enzym Adenylcyclase gehemmt. Adachi (1970) hat herausgefunden, dass die Aktivität von Adenylcyclase bzw. die Konzentration an cyclo-Adenosin-Monophosphat (cAMP) eng mit dem Haarwachstum verknüpft ist. cAMP ist bekannt als second-messenger und aktiviert sowohl die Energiegewinnungskaskaden als auch die Zell-Neogenese durch Aktivierung des Protcin-Kinase-Systems. Beide Stoffwechselwege sind für den normalen gesunden Haarwuchs von Bedeutung. Unter einer Mangelsituation lcitct der aktive Haarfollikel spontan eine Ruhephase ein, in der das betroffene Haar vorzeitig ausfällt.

Nach einer mehrmonaligen Ruhephase wächst aus dem Haarfollikel zwar wieder ein neues Haar. Da der Mangelzustand wegen der unverändert fort bestehenden genetisch bedingten Überempfmdlichkeit andauert, kann auch die neue Haarwachstumsphase nicht normal beendet werden. In Folge verkürzen sich die Wachstumszyklen und der Haarfollikel verkümmert und erschöpft schließlich.

Unter androgenetisch bedingtem Haarausfall leiden sowohl Männer als auch Frauen. Obwohl Frauen in der Regel erst mit Beginn der Menopause zu dem Kreis der unmittelbar Betroffenen zählen, ist das Maß an Betroffenheit bei Frauen ungleich höher als bei Männern.

Durch das Absinken des Östrogenspiegels im Verlauf der Menopause entsteht ein hormonelles Unglcichgcwicht zugunsten von Testosteron, so dass die genetisch bedingte Veranlagung in den Haarwurzeln zum Ausdruck kommt. Das Haar wird dünner und fällt vorzeitig aus. Meist beobachtet man nicht das typische Muster bzw. die typischen Verlaufsformen wie beim Mann. Bei der Frau tritt der Haarausfall eher in diffuser Form auf.

Das Übergewicht an Testosteron bzw. der Mangel an Östrogenen führt bei Frauen aber nicht nur zu Haarproblemen, sondern auch zu zahlreichen weiteren Beschwerden, wie z.B. depressiven Stimmungen, vegetativen Dysfunktionen (Hitzewallungen, Schweißausbrilche), etc. Deshalb hat man schon früher versucht, diese menopausalen Beschwerden zu beseitigen. Zuerst wollte man den Mangel an Östrogenen durch eine externe Zufuhr von östrogenen Hormonen behandeln. In der Tat gelang es rasch, die Beschwerden zu beseitigen. Diese Therapieform wird heute jedoch nicht mehr uneingeschränkt empfohlen, weil bekannt ist, dass durch hohe Östrogenspiegel im Alter das Risiko von Mamma-Karzinomen deutlich ansteigt. Beim Mann war die Östrogenbehandlung bei androgenetisch bedingtem Haarausfall zwar auch erfolgreich, wurde jedoch wegen unerwünschter Nebenwirkungen nie etabliert.

In den letzten Jahren hat sich in der Behandlung menopausaler Beschwerden die Östrogenersatz-Therapic gut eingeführt. Man hat erkannt, dass durch bestimmte pflanzliche Isoflavone, z.B. aus Soja (Genistein), Olive (Oleuropcin), Actein/Cimigosid aus der Traubensilberkerze und Rotklee-Extrakt ein Östrogenmangel behoben werden kann, ohne die bereits beschriebenen Risiken in Kauf zu nehmen. Diese sogenannten Yhyto-Östrogene lösen an bestimmten Rezeptoren östrogenähnliche Effekte aus. So werden beispielsweise in der US 6,338,855 A Extrakte aus der Traubensilberkerze zur Behandlung von Hautatrophien vorgeschlagen, die US 6,153,208 nennt deren Verwendung als antiphlogistisches Wirkprinzip in der Haut.

Wegen der guten Erfahrungen in der Östrogenersatz-Therapie hat man diese Pflanzenextrakte auch vereinzelt zur Behandlung des androgenetischen Haarausfalles vorgeschlagen. So wird beispielsweise in der CA 2,132,170 Olivenöl zur Behandlung von Alopezie vorgeschlagen.

Es zeigte sich jedoch, dass zumindest die Eigenschaften der antiandrogenen Effekte bei der ausschließlichen Anwendung von Phytoöstrogenen nicht besonders ausgeprägt sind. Dies mag daran liegen, dass die Penetration mancher biologisch aktiver Extrakte durch die Haut nicht in ausreichendem Maße stattfindet oder aber die Voraussetzungen für deren Wirksamkeit in der Haarwurzel sind nicht voll entfalten.

Aufgabe der Erfindung ist es somit, bei einem Mittel zur Aktivierung der Haarwurzeln die Wirkung der "östrogenen" Pflanzenextrakte noch zu verstärken.

Diese Aufgabe wird erfindungsgemäß dadurch gelöst, dass das Mittel aus einer Kombination von Coffein und einem. Pflanzenextrakt mit phytoöstrogenen Eigenschaften besteht.

Überraschenderweise wurde gefunden, dass sich die den Haarwuchs fördernden Eigenschaften durch den Zusatz von Coffein deutlich steigern ließen. Coffein ist eine Substanz, die aufgrund der geringen Molekülgröße in die Haut pcnctrieren kann. Interessanterweise ist der Haarwuchs kräftigende Effekt durch die Mischung der beiden Wirkprinzipien besser als er durch Einzelkomponenten zu erwarten wäre.

Coffein besitzt eine ZNS-stimulierende Wirkung, die offensichtlich auch bei vorzeitigem bzw. androgenetischem Haarausfall genutzt werden kann. In Verbindung mit den genannten pflanzlichen Flavonen kann eine deutliche Reduzierung des Haarausfalles festgestellt werden. Coffein besitzt auch eine durchblutungsfordernde Eigenschaft, weshalb es ebenfalls schon in verschiedenen Schriften zur Behandlung von Haarausfall genannt wurde. Beispielhaft sei genannt die DE 197 01 651 A1, in welcher Coffein als Bestandteil einer Rezeptur zur Behandlung von Haarausfall genannt wird. Der synergistische Effekt wird dahingehend interpretiert, dass durch Stoffwechselanregung des Coffeins in der Haarwurzel die Wirkung der Stimulation von Östrogen-Rezeptoren hier durch die Pseudoöstrogene aus der Traubensilberkerze (Cimicifuga racemosa) deutlich gesteigert werden kann.

Es liegt im Rahmen der Erfindung, dass das Coffein ein Extrakt aus einer oder mehreren der Pflanzen Kaffeebohne (Coffea arabica, canephora, liberica, dewevrei), Teestrauch (Camellia sinensis), Cocapflanze (Cola nitida), Matepflanze (Ilex paragueriensis), Kakaopflanze (Theobroma cacao) und Guaraná (Paullinia cupana) ist. Auch synthetische Gewinnungsweisen des Coffeins sind bekannt und können genutzt werden.

Weiterhin ist vorgesehen, dass der Pflanzenextrakt mit phytoöstrogenen Eigenschaften ein Extrakt aus einer oder mehreren der Pflanzen Soja (Glycine soja), Olive (Olea europaea), Rotklee (Trifolium pratense), Kürbis (Cucurbita) und Traubensilberkerze (Cimicifuga racemosa) ist.

Es ist zweckmäßig, dass der Coffeingehalt zwischen 0,01 und 50% liegt.

Eine Weiterbildung der Erfindung besteht darin, dass das Mittel zusätzlich amphotere oder nichtionische oberflächenaktive Stoffe enthält.

Hierbei ist vorgesehen, dass der Gehalt an amphoteren oder nichtionischen oberflächenaktiven Stoffen zwischen 0,5 und 10% beträgt.

Ebenso ist es möglich, dass das Mittel 0,1 bis 50%, vorzugsweise 1 bis 15% anionische Tenside enthält.

Eine Weiterbildung der Erfindung besteht darin, dass das Mittel Proteinzusätze, Aminosäuren, Spurenelemente und/oder Vitamine enthält.

Es kann auch vorteilhaft sein, dass das Mittel die Haarwurzel stabilisierende Stoffe, insbesondere Minoxidil und/oder Finasterid enthält.

Im Rahmen der Erfindung liegt auch, dass das Mittel Antischuppenwirkstoffe, insbesondere Piroctone Olamine, Zink-Pyrithion, Climbazol, Salicylsäure und/oder Fumarsäure enthält.

Es ist zur Erfindung gehörig, dass das Mittel als Shampoo-Formulierung zusammengesetzt ist.

Eine andere Ausgestaltung der Erfindung besteht darin, dass das Mittel als wässriges oder wässrig-alkoholisches Tonikum zusammengesetzt ist.

Ebenso kann es zweckmäßig sein, dass das Mittel als Spülungsformulierung mit kationischen Haarpflegestoffen zusammengesetzt ist.

Erfindungsgemäß ist auch vorgesehen, dass das Mittel als Haar- oder Kopfhautkur mit Lipidstoffen zur Pflege trockener Haare und Kopfhaut zusammengesetzt ist.

Es kann auch sinnvoll sein, dass das Mittel entweder als Schaum oder als schäumendes Gel vorliegt und als Aerosolanwendung künditioniert ist.

Ebenso ist es denkbar, dass das Mittel als Spray oder als Pumpspray vorliegt.

Eine andere Möglichkeit besteht darin, dass das Mittel als 2-Komponentenpackung aus einer Produktgrundlage und einem Wirkstoffkonzentrat vorliegt.

Im Rahmen der Erfindung liegt auch die Verwendung des erfindungsgemäßen Mittels zur Vorbeugung gegen erblich bedingten Haarausfall oder zur Vermeidung dünner, brüchiger Haare.

Das erfindungsgemäße Stoffgemisch kann als Rezeptur angewendet werden, die in ständigem Kontakt mit der Kopfhaut bleibt. Solche Leave-On-Präparate haben den Vorteil, dass sie ein Wirkstoff-Depot auf der Haut darstellen und über eine lange Zeit eine Schutzwirkung entfalten können. In der Regel erzielt man damit eine besonders gute und nachhaltige Wirksamkeit. Leave-On-Präparate können als Konzentrat im Sinne einer Ampulle, als wässrige oder wässrig-alkoholische Lösung, als Pflegeemulsion oder als Hydro-Gel formuliert werden. Gerade die leicht viskosen Formulierungen sind zweckmäßig, weil sie konzentriert auf der Kopfhaut verbleiben und weniger stark in den Haarschaft spreiten können. Allerdings ist bei diesen Formulierungen zu beachten, dass die Haare durch die Konsistenz gebenden Stoffe nicht belastet werden, da diese dann einen etwas ungepflegten Eindruck hinterlassen. Deshalb kann es auch vorteilhaft sein, wenn diese Haarausfall vorbeugenden Rezepturen als Rinse-Off-Präparat in Form eines Shampoos oder einer Kopfhaut-/Haar-Kur oder ähnlichem angewendet werden. Der Vorteil ist, dass die Belastung des Haares nicht so ausgeprägt ist, weil diese Mittel wieder aus dem Haar ausgespült werden. Allerdings ist zu beachten, dass bei den Anwendungen als Rinse-Off Formulierung eine Einwirkzeit von ca. 2-5 min vorzusehen, damit die notwendige Penetration in die Haarwurzeln möglich wird. Grundsätzlich ist aus verschiedenen Untersuchungen auch bei kurzem Kontakt mit der Kopfhaut schon eine wesentliche Penetration zu erwarten, da durch Penetrationshilfsstoffe die biologische Verfügbarkeit der aktiven Stoffe maßgeblich verbessert wird.

Der erfindungsgemäße Gegenstand kann deshalb durch zusätzliche Wirk- und Hilfsstoffe ergänzt werden. Die Aufzählungen nachfolgender Stoffe ist nicht vollständig, sondern nur beispielhaft, um zu erklären, in welchem Zusammenhang der erfindungsgemäße Gegenstand anwendbar ist. Grundsätzlich sind damit auch andere nicht genannten Stoffe eingeschlossen, sofern sie vergleichbare Stoffeigenschaften aufweisen.

Als Penctrationshilfsstoffe kommen anionische Tenside in Form von folgenden Stoffgruppen in Frage: Alkylothersulfate, Alkylsulfate, Alkyl-Sulfosuccinate, Ethercarbonsäuren oder Estercarbonsäuren, Alkylsarkosinate, -glutamate, - glycinate und -taurate oder Alkylphosphate. Ergänzt werden die anionischen Tenside durch amphotere Tenside, Alkylamidopropylbetain wie z.B. Cocamidopropylbetain, Alkylamphoactetate oder -diacetate, wie z,B, Cocoamphoactetate, Cocamide MEA oder -DEA und nichtionische Tenside. Beispielhaft sind aufgezählt Alkylglycoside und Alkoxyglycoside.

Insbesondere sind Kombinationen mit sekundären Pflanzeninhaltsstoffen aus Ghamomilla, Hypericum, Ginkgo, Tussilago, Berberis, Camellia, Glycyrrhiza, Humulus möglich, die reizlindernd und antiirritativ wirksam sind. Ebenso sind Zusätze aus löslich gemachten Proteinfraktionen pflanzlicher oder tierischer Quellen möglich. Die Zusätze können aus Weizen, Hafer, Seide, Kollagen, Keratin, Milchprotein, Soja, biotechnologisch gewonnenem Pseudokollagen etc. stammen. Sie werden eingesetzt, um Strukturschäden am Haar zu beseitigen oder als sogenannte Feuchthaltestoffe. Ergänzend können weitere Feuchthalte-Stoffe wie Panthenol, Allantoin, Glycerin, Glycole, Sorbit, Harnstoff, Polyglycole oder Polyglycerine eingesetzt werden.

Vorteilhaft für die Pflege der Kopfhaut sind Zusätze von Vitaminen, z.B. Niacin, Biotin, Vitamin E, Vitamin A, Vitamin C oder Coenzym Q10, auch deren Derivate, insbesondere Ester der oxidationsempfindlichen Vitamine E und A.

Zur gleichzeitigen Beseitigung von Kopfschuppen kann Piroctone Olamine, Salicylsäure, Fumarsäure, Climbazol oder Zink-Pyrithion eingesetzt werden.

Zur Verbesserung des Haargeruches und wegen des erfrischenden und durchblutungsfördernden Effektes können Terpene eingesetzt werden, wie z.B. Farnesol, Menthol Thymol etc. Auch Ester von Terpenalkoholen mit organischen Säuren können eingesetzt werden, damit ein retardierender Effekt in der Kopfhaut erzielt werden kann.

Zur Haarpflege insbesondere poröser Haare können kationische Pflegestoffe in monomerer als auch polymerer Form vorliegen. Beispielhaft sei hier genannt Cetrimoniumchlorid , als polymerer Pflegestoff gilt z.B. Polyquaternium-10.

Einen ebenfalls positiven Effekt auf die Pflege pöröser Haaroberflächen und trockener, schuppender Kopfhaut haben Fettalkohle sowie deren Esterverbindungen mit Fett- oder Mineralsäuren oder Etherverbindungen von Fettalkoholen mit ein oder mehrwertigen Alkoholen, die wiederum selbst zur Gruppe von Fettalkoholen oder aber auch Zuckeralkoholen zählen. Zur besseren Solubilisierung können diese Fettalkohole auch gemischte Polyetherverbindungen sein, die sich aus einer Teilkette in Form von Polyethoxylaten oder Polyglyceraten und Fettalkoholen darstellen.

Lichtschutzfilter, um das Haar vor UV-Strahlung zu schützen, sind ebenfalls von Nutzen. Als Vertreter dieser Wirkstoffgruppe gelten Benzophenone, p-Aminobenzoesäure, Zimtsäurederivate, bis-t-Butyl-Dibenzoylmethan, 4-Methyl-Benzylidencampher.

Auch pflanzliche oder synthetische Öle zur Verbesserung des Glanzes und der Geschmeidigkeit des Haares sind nützlich. Typische pflanzliche Öle sind Sojaöl, Sonnenblumenöl, Mandelöl, Traubenkernöl, Avocadoöl, Jojobaöl, aus synthetischer Herkunft moditizierte Silikonöle des Typs Dimethicone und Dimethiconol, auch als kationische Derivate wie Amodimethicone. Weitere synthetische Öle, Fette oder Wachse sind Paraffinöle oder längerkettige Fraktionen von Kohlenwasserstoffen wie Petrolatum oder Ceresine. Weitere Kopfhaut- und Haarpflegestoffe natürlicher Herkunft sind Squalen, Sterine pflanzlicher oder tierischer Herkunft, sowie hydrierte Derivate dieser Stoffe.

Zur Stabilisierung werden Komplexbildner wie NTA, EDTA, Phytinsäure, Polyphosphate, HEDP, Citronensäure, Weinsäure und andere Polyhydroxycarbonsäuren eingesetzt. Weitere Stabilisatoren sind Antioxidalionsmittel wie BHT, BHA, Vitamin E Acelat.

Zur Vermeidung mikrobiollen Verderbs bei der Anwendung werden organische Säuren und deren Salze wie Ameisensäure, Sorbinsäure oder Benzoesäure vorgeschlagen oder Ester der p-Hydroxybenzoesäure, Formaldehyd abspaltende Konservierungsstoffe wie DMDM Hydantoin, Imidazolidinylharnstoff, oder Methylchloroisothiazolinon, Methylisothiazolinone, Dibromodicyanobutane, lodopropynyl Butylcarbamate, Phenoxyethanol oder Benzylakohol.

Zur Stabilisierung des pH-Wertes werden Puiffersalze mehrwertiger organischer Säuren wie Citronensäure, Weinsäure oder Äpfelsäure eingesetzt.

Zur Verbesserung der Konsistenz haben sich nichtionische Verdicker, sogenannte Alkylethoxylate bewährt. Abschließend ohne Nennung detaillierter Beispiele soll nicht unerwähnt bleiben, dass zur Solubilisierung wasserunlöslicher Wirkstoffe auch ein- oder mehrwertige Alkohole eingesetzt werden können, ebenso Methyl- oder Ethylether mehrwertiger Alkohole oder Polyglycerine oder Polyglycole.

Die erfindungsgemäßen Stoffe können auch zur Verbesserung der biologischen Verfügbarkeit in sogenannten liposomalen Vesikeln eingeschlossen sein. Andere Transportsysteme können Nanosomen, Mikrosomen, Mikrospheren oder Niosomen sein. Auch Einschlussverbindungen wie Cyclodextrine können einen die Wirkung steigernden Effekt erzielen.

Ein Tonikum wird üblicherweise als wässrig-alkoholische Lösung formuliert, wobei der Alkohol sowohl Ethanol als auch iso-Propanol oder n-Propanol sein kann.

Die vorgeschlagenen Formulierungen können entweder als Tonikum mittels Dosierspitze direkt auf die Kopfhaut aufgetragen werden; sie können auch über einen Dosierspender appliziert werden. Zur besseren Feinverteilung dünnflüssiger Systeme eignen sich Sprühapplikatoren, die wiederum entweder durch Treibgas unterstützt werden oder aber nur durch verdichtende Pumpsysteme. Eine Applikation höher viskoser Formulierungen aus einer Tube ist ebenso zweckdienlich wie die Anwendung aus einem Tiegel oder einem Spender bei cremigen Zubereitungen.

Ebenfalls zur Verbesserung der Verteilung auf der Kopfhaut können Acrosolschäume oder Pumpschäume in Betracht kommen.

Sind einzelne Komponenten in der Mischung nicht stabil und zersetzen sich bei Lagerung, so besteht die Möglichkeit, diese erst unmittelbar vor der Anwendung zu mischen. Dafür sind sogenannte 2-Komponenten-Packungen üblich, die entweder ein flüssiges oder ein pulverförmiges bzw. kristallines Konzentrat enthalten.

Die beschriebenen Verpackungsformen sollen eine möglichst optimale Anwendung des erfindungsgemäßen Gegenstandes bewirken.

Die dafür in Frage kommenden Rezepturgrundlagen sind tonisierende Mittel, sogenannte Wässer oder Liquids, dünnflüssige Emulsionen wie z.B. Lotionen, in höherer Konsistenz Spülungen, Haarkuren, Haareremes oder Haarmasken, auch Hydrogele und nicht zuletzt waschaktive Zubereitungen wie Shampoos sind typische Rezepturformen, über die der erfindungsgemäße Gegenstand appliziert werden kann. Der Vorteil liegt hier bei der heutzutage sehr häufigen Anwendung. Somit können der Kopfhaut regelmäßig die erfindungsgemäßen Schutzstoffe zugeführt werden.

Vielfach werden auch unterschiedliche Wirkungen in den Rezepturen kombiniert, um dem Anwender den größtmöglichen Nutzen zu verschaffen. So sind als "Zwei in Eins"- oder "Drei in Eins"-Produkte Kombinationen von Haarreinigung und Haarpflege ebenso vorstellbar wie Kombinationen aus Antischuppen-Wirkstoffen und den Haarwuchs steigernden Wirkstoffkomplexen.

Werden Haarkuren als Rezepturgrundlage bzw. Wirkstoffmatrix des erfindungsgemäßen Gegenstandes eingesetzt, so ist hier durch die etwas längere Einwirkzeit sogar noch eine verstärkte Wirkung zu erwarten. Der Einsatz in einer Kur ist auch insofern vorteilhaft, weil der Kunde einen erkennbaren Zusatznutzen erzielt. Da Kuren bisher in der Regel zur Verbesserung der Struktur des äußeren IIaarschaftes eingesetzt werden, könnte durch die kombinierte Haar- und Kopfhaut-Kur eine Verbesserung der Haarbeschaffenheit von Grund auf durch die Stabilisierung der Haarwurzel erzielt werden.

Zur Verdeutlichung der Vielfalt an Rezepturvarianten sollen nachfolgende Rahmenrezepturcn dienen;

| Formel A Haarshampoo | Gew.-% |
|---|---|
| Sodium Laureth Sulfate | 10.00% |
| Cocoamphoacetate | 2.00% |
| Glycerin | 2,00% |
| Panthenol | 0.5.% |
| Hydrolyzed Collagen | 1.00% |
| Trifoilium Pratense | 0,40% |
| Sodium Polyphosphate | 0,10% |
| Parfum | 0.30% |
| Sodium Chloride | 0.30% |
| Cimicifuga Racemosa | 0.50% |
| Gaffeine | 0.30% |
| Formic Acid | 0,20% |
| Aqua | ad 100 |

| Formel B Haarshampoo für feines Haar | Gew.-% |
|---|---|
| Sodium Lauryl, Sulfate | 4,00% |
| Sodium Laureth Sulfate | 5,00% |
| Hydroxypropyltrimonium Hydrolyzed Wheat | 1.00% |
| Disodium Laurcth Sulfosuccinate | 3.00% |
| Biotin | 0.02% |
| Tocopherylacetate | 0.30% |
| Citric Acid | 0.20% |
| Caffeine | 0.50% |
| Parfum | 0.30% |
| Potassium Sorbate | 0,20% |
| DMDM Hydantoin | 0,10% |
| Aqua | ad 100 |

| Formel C Tonikum | Gew.-% |
|---|---|
| Alcohol Denat | 40.00 |
| Tocopherylacetat | 0,05% |
| PEG-40 Hydrogenated Castor Oil | 0.30% |
| Caffeine | 0.30% |
| Glycine Soya | 0,20% |
| Panthenol | 0.30% |
| Parfum | 0.20% |
| Menthol | 0,10% |
| Aqua | ad 100 |

| Formel D "Zwei in Eins" - Tonikuin | Gew.-% |
|---|---|
| Alcohol Denat | 45.00% |
| Piroctone Olamine | 0,20% |
| Salicylic Acid | 0,20% |
| Caffeine | 0,20% |
| Olea Europaea | 0,30% |
| Biotin | 0,01% |
| Hydrolyzed Keratin | 0,40% |
| Aqua | ad 100 |

| Formel E Haarspülung | Gew.-% |
|---|---|
| Cetrimonium Chloride | 2,00% |
| Steareth-10 | 5,00% |
| Cetearyl Acohol | 4,00% |
| Parfum | 0,50% |
| Caffeine | 0,40% |
| Hydrolized Keratin | 0,50% |
| Citric Acid | 0,20% |
| Sodium Benzoate | 0,05% |
| Cimicifuga Racemosa | 0,50% |
| Potassium Sorbatc | 0,15% |
| Aqua | ad 100 |

| Formel F Haar- und Kopfhaut-Kur | Gew.-% |
|---|---|
| Cetyl Alcohol | 6,00% |
| Panthenol | 1,00% |
| Caffeine | 0,50% |
| Trifolium Pratense | 1,00% |
| Polyquaternium-10 | 0,50% |
| Dimethyconol | 2,00% |
| Phytic Acid | 0,50% |
| Bisabolol | 0,20% |
| Hydrolized Milk Protein | 0,50% |
| PEG-25 PABA | 0,50% |
| Aqua | ad 100 |

## Patentansprüche

1. Mittel zur Aktivierung der Haarwurzeln, **dadurch gekennzeichnet, dass** das Mittel aus einer Kombination von Coffein und einem Pflanzenextrakt mit phytoöstrogenen Eigenschaften besteht.

2. Mittel gemäß Anspruch 1, **dadurch gekennzeichnet, dass** das Coffein ein Extrakt aus einer oder mehreren der Pflanzen Kaffeebohne (Coffca arabica, canephora,, liberica, dewevrei), Teestrauch (Camellia sincnsis), Cocapflanze (Cola nitida), Matepflanze (IIex paragueriensis), Kakaopflanze (Theobroma cacao) und Guaraná (Paullinia cupana) ist.

3. Mittel gemäß Anspruch 1, **dadurch gekennzeichnet, dass** das Pflanzenextrakt mit phytoöstrogenen Eigenschaften ein Extrakt aus einer oder mehreren der Pflanzen Soja (Glycine soja), Olive (Olea europaea), Rotklee (Trifolium pratense), Kürbis (Cucurbita) und Traubensilberkerze (Cimicifuga racemosa) ist.

4. Mittel gemäß Anspruch 1, **dadurch gekennzeichnet, dass** der Coffeingehalt zwischen 0,01 und 50% liegt.

5. Mittel gemäß Anspruch 1, **dadurch gekennzeichnet, dass** das Mittel zusätzlich amphotere oder nichtionische oberflächenaktive Stoffe enthält.

6. Mittel gemäß Anspruch 5, **dadurch gekennzeichnet, dass** der Gehalt an amphoteren oder nichtionischen oberflächenaktiven Stoffen zwischen 0,5 und 10% beträgt.

7. Mittel gemäß Anspruch 1, **dadurch gekennzeichnet, dass** das Mittel 0,1 bis 50%, vorzugsweise 1 bis 15% anionische Tenside enthält.

8. Mittel gemäß Anspruch 1, **dadurch gekennzeichnet, dass** das Mittel Proteinzusätze, Aminosäuren, Spurenelemente und/oder Vitamine enthält.

9. Mittel gemäß Anspruch 1, **dadurch gekennzeichnet, dass** das Mittel die Haarwurzel stabilisierende Stoffe, insbesondere Minoxidil und/oder Finasterid enthält.

10. Mittel gemäß Anspruch 1, **dadurch gekennzeichnet, dass** das Mittel Antischuppenwirkstoffe, insbesondere Piroctone Olamine, Zink-Pyrithion, Cilmbazol, Salicylsäure und/oder Fumarsäure enthält.

11. Mittel gemäß einem der Ansprüche 1 bis 4 oder 8 bis 10, **dadurch gekennzeichnet, dass** das Mittel als Shampoo-Formulierung zusammengesetzt ist.

12. Mittel gemäß einem der Ansprüche 1 bis 10, **dadurch gekennzeichnet, dass** das Mittel als wässriges oder wässrig-alkoholisches Tonikum zusammengesetzt ist.

13. Mittel gemäß einem der Ansprüche 1 bis 10, **dadurch gekennzeichnet, dass** das Mittel als Spülungsformulierung mit kationischen Haarpflegestoffen zusammengesetzt ist.

14. Mittel gemäß einem der Ansprüche 1 bis 10, **dadurch gekennzeichnet, dass** das Mittel als Haar- oder Kopfhautkur mit Lipidstoffen zur Pflege trockener Haare und Kopfhaut zusammengesetzt ist.

15. Mittel gemäß einem der Ansprüche 11 bis 14, **dadurch gekennzeichnet, dass** das Mittel entweder als Schaum oder als schäumendes Gel vorliegt und als Aerosolanwendung konditioniert ist.

16. Mittel gemäß einem der Ansprüche 11 bis 14, **dadurch gekennzeichnet, dass** das Mittel als Spray oder als Pumpspray vorliegt.

17. Mittel gemäß einem der Ansprüche 11 bis 14, **dadurch gekennzeichnet, dass** das Mittel als 2-Komponentenpackung aus einer Produktgrundlage und einem Wirkstoffkonzentrat vorliegt.

18. Verwendung des Mittels gemäß den Ansprüchen 1 bis 17 zur Vorbeugung gegen erblich bedingten Haarausfall.

19. Verwendung des Mittels gemäß den Ansprüchen 1 bis 17 zur Vermeidung dünner, brüchiger Haare.
